# EUROPEAN PATENT SPECIFICATION

(11) **EP 2 737 859 B1**
(45) Date of publication and mention of the grant of the patent: **06.11.2019**
(21) Application number: 14157167.9
(22) Date of filing: 14.09.2010
(51) Int. Cl.: A61B 8/12, A61B 8/00

(54) **Image diagnosis device**
BILDDIAGNOSEVORRICHTUNG
Dispositif de diagnostic d'image

(30) Priority: 30.09.2009 JP 2009227842
(43) Date of publication of application: 04.06.2014
(62) Divisional of application: 10820081.7
(73) Proprietor: Terumo Kabushiki Kaisha, Tokyo (JP)
(72) Inventor: Irisawa, Yuichiro, KANAGAWA, 259-0151 (JP)
(74) Representative: Casalonga

(56) References cited:
- DE-A1-102007 043 639
- DE-U1- 9 400 080
- JP-A- 2002 034 981
- US-A- 5 734 219
- US-A1- 2008 177 183
- US-A1- 2009 129 555

## Description

### TECHNICAL FIELD

The present invention relates to an imaging apparatus for diagnosis, in particular to an intravascular imaging apparatus according to claim 1, such as it is known for example from US20080177183.

### BACKGROUND ART

From the past, there have been widely used an intravascular ultrasound (IVUS: Intra Vascular Ultra-Sound) apparatus for diagnosis for a diagnosis before operation at the time of treatment inside a blood vessel depending on a high functional catheter such as a balloon catheter, a stent and the like or for a result confirmation after operation. Also, recently, there has been progressed the development of an intravascular optical coherent tomographic imaging (OCT, OFDI) apparatus for diagnosis using an optical coherent tomography method, which has higher resolution (for example, see Japanese Patent No. 4037538). Note that hereinafter, those intravascular ultrasound (IVUS) apparatus for diagnosis and intravascular optical coherent tomographic imaging (OCT, OFDI) apparatus for diagnosis will be generically referred to as "imaging apparatus for diagnosis".

Within the imaging apparatuses for diagnosis, the IVUS apparatus for diagnosis uses ultrasound waves, so that there is such an advantage that the attenuation inside the living body is small and a tomographic image is obtained over a wide range. On the other hand, the OCT and OFDI image apparatuses for diagnosis use laser, so that while they have such an advantage that a tomographic image of higher resolution compared with that of the IVUS apparatus for diagnosis can be obtained, attenuation in the living body is large and therefore, they have such a defect that it is possible to obtain only a tomographic image in a narrow range.

Consequently, there is differently used an IVUS apparatus or an OCT (or OFDI) apparatus at a medical site depending on the diagnosis object and in a case in which it is necessary to use both of them at a time, a situation is to be applied in which there are utilized a probe including a transmitting and receiving unit for the IVUS apparatus and a transmitting and receiving unit for the OCT (or OFDI) apparatus (hereinafter, referred to as a compatible probe) and a scanner & pull-back unit to which the aforesaid compatible probe is connected detachably.

Here, an IVUS apparatus and a OCT (or OFDI) apparatus are common in an aspect that tomographic images inside and outside a blood vessel are generated by radially scanning a transmitting and receiving unit in a state in which a probe is inserted into the blood vessel.

Consequently, it is necessary for a scanner & pull-back unit, which is attached and detached with a compatible probe unit, to be arranged usually with two kinds of rotary connectors (joints). Specifically, they are a metal contact type rotary connector for an IVUS apparatus, which is used for an electric signal transmission and a non-contact type optical rotary connector for an OCT or OFDI apparatus, which is used for an optical signal transmission.

In other words, for the scanner & pull-back unit with which a compatible probe is connected detachably, it becomes a situation in which a metal contact type rotary connector and a non-contact type optical rotary connector coexist.

### DISCLOSURE OF THE INVENTION

### [Problem to be solved by the Invention]

Here, in case of the IVUS apparatus, it is possible to generate a tomographic image without being influenced by the blood, so that a tomographic image is generated by a frame rate of around 30 frames/second. On the other hand, in case of the OCT or OFDI apparatus, it happens that the laser is scattered & absorbed by the blood, so that it is necessary to substitute the blood by a physiological saline or the like beforehand during a time when a tomographic image is generated and the inside of the blood vessel becomes in an ischemic state during the time when the tomographic image is generated. Consequently, in case of the OCT or OFDI apparatus, it is necessary to generate the tomographic image by a frame rate of higher speed than that of the IVUS apparatus (that is, it is necessary to rotate the transmitting and receiving unit in a high speed).

Consequently, as described above, in the scanner & pull-back unit connected with the compatible probe, it happens, while using the OCT or OFDI apparatus, that the metal contact type rotary connector rotates in a high speed (compared with a case using the IVUS apparatus), so that it causes breakdown and/or short lifetime due to the occurrence of abrasion powders or the like.

The present invention was invented in view of the problem mentioned above and is addressed to reduce the wear of the metal contact type rotary connector in an imaging apparatus for diagnosis provided with a metal contact type rotary connector for electric signals and a non-contact type rotary connector for optical signals.

### [Means for solving the Problem]

In order to achieve the object mentioned above, there is provided an imaging apparatus according to claim 1. The dependent claims relate to advantageous embodiments.

### [Effect of the Invention]

According to the present invention, it becomes possible to reduce the wear of the metal contact type rotary connector in the imaging apparatus for diagnosis provided with a metal contact type rotary connector for an electric signal and a non-contact type rotary connector for an optical signal.

Other features and advantages of the present invention will become clear according to the following explanations with reference to the attached drawings. Note that in the attached drawings that identical reference numbers are to be attached for the identical or similar constitutions.

### BRIEF DESCRIPTION OF DRAWINGS

The attached drawings are included in the specification, constitute a portion thereof, show illustrastive examples not falling under the scope of the claims and exemplified embodiments of the present invention, and are used together with the description thereof for explaining principles of the present invention.
FIG. 1 is a diagram showing an outward-appearance constitution of an optical imaging apparatus for diagnosis relating to a first illustrastive examples not falling under the scope of the claims;
FIG. 2 is a diagram showing a constitution of a probe unit;
FIG. 3 is a diagram showing a constitution of an imaging core inside the probe unit;
FIG. 4 is a diagram showing an inside constitution on the proximal end side of a drive shaft connector in the probe unit;
FIG. 5 is a diagram showing a constitution of an optical adaptor and a rotary connector of a scanner unit;
FIG. 6A is a diagram showing a constitution of the electric rotary connector;
FIG. 6B is a diagram showing a constitution of the electric rotary connector;
FIG. 7 is a diagram showing an aspect in which the probe unit and the scanner unit are connected;
FIG. 8 is a diagram showing an inside constitution on the proximal end side of the drive shaft connector in the probe unit;
FIG. 9A is a diagram showing a constitution of the electric rotary connector;
FIG. 9B is a diagram showing a constitution of the electric rotary connector;
FIG. 10 is a diagram showing a constitution of the optical adaptor and the rotary connector of the scanner unit;
FIG. 11 is a diagram showing an aspect in which the probe unit and the scanner unit are connected;
FIG. 12 is a diagram showing a constitution of the optical adaptor and the rotary connector of scanner unit;
FIG. 13A is a diagram showing a constitution of the electric rotary connector;
FIG. 13B is a diagram showing a constitution of the electric rotary connector; and
FIG. 14 is a diagram showing a functional block of a brushing operation control.

### MODE FOR CARRYING OUT THE INVENTION

Hereinafter, it will be explained with respect to respective illustrative examples not falling under the scope of the claims and exemplified embodiments of the present invention in detail with reference to the attached drawings if necessary.

### [First illustrative example not falling under the scope of the claims]

### <1. Outward-Appearance Constitution of Imaging Apparatus for Diagnosis>

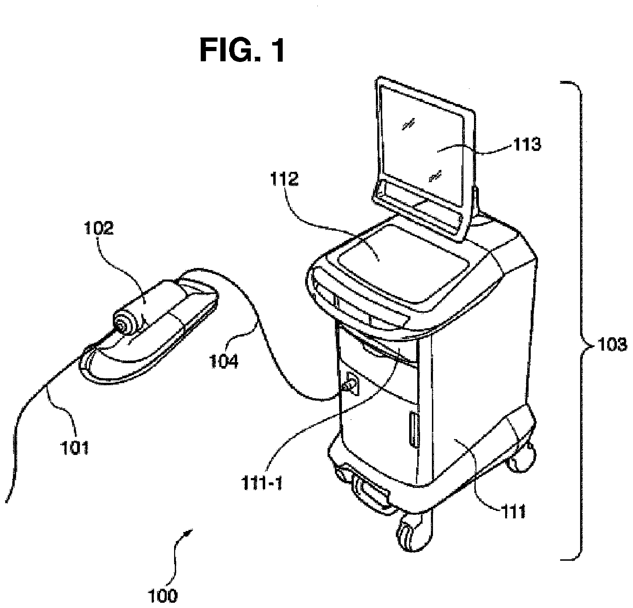

FIG. 1 is a diagram showing an outward-appearance constitution of an imaging apparatus for diagnosis (intravascular ultrasound (IVUS) apparatus for diagnosis, optical coherent tomography (OCT) apparatus or optical frequency domain imaging (OFDI) apparatus for diagnosis utilizing wavelength sweep) 100 relating to a first illustrative example not falling under the scope of the claims.

As shown in FIG. 1, the imaging apparatus for diagnosis 100 is provided with a probe unit 101, a scanner & pull-back unit 102 and an operation control apparatus 103, and the scanner & pull-back unit 102 and the operation control apparatus 103 are connected by means of a signal line 104.

The probe unit 101 is inserted directly into the inside of blood vessel and measures the state inside the blood vessel by obtaining a reflected signal inside the blood vessel by using an imaging core including a transmitting and receiving unit for IVUS and a transmitting and receiving unit for OCT (or OFDI) (details will be described later).

The scanner & pull-back unit 102 is a unit constituted integrally by a scanner unit defining the rotation operation of the imaging core and a pull-back unit defining the linear operation of the imaging core, and it defines the radial and axial operations of the imaging core by a configuration in which the scanner unit and the pull-back unit operate in parallel.
within those units, it is possible for the scanner unit to be connected detachably with the probe unit 101 without using a special tool and in the inside thereof, there are included a metal contact type rotary connector for electric signal (electric rotary connector) and a non-contact type rotary connector for optical signal (optical rotary connector).

The operation control apparatus 103 is provided with a function for inputting various kinds of set values on an occasion when carrying out intravascular tomographic diagnosis, and a function for processing data obtained by the measurement and for generating and displaying tomographic images.

In the operation control apparatus 103, a reference numeral 111 indicates a main body control unit, and it processes data obtained by the measurement, outputs the processed result and so on. A reference numeral 111-1 indicates a printer & DVD recorder and the processed result in the main body control unit 111 is printed, is stored as data signals and so on.

A reference numeral 112 indicates an operation panel (interface) and a user carries out input of various kinds of set values through the operation panel 112. A reference numeral 113 indicates an LCD monitor as a display apparatus and it displays the processed result in the main body control unit 111.

### <2. Whole Constitution of Probe Unit>

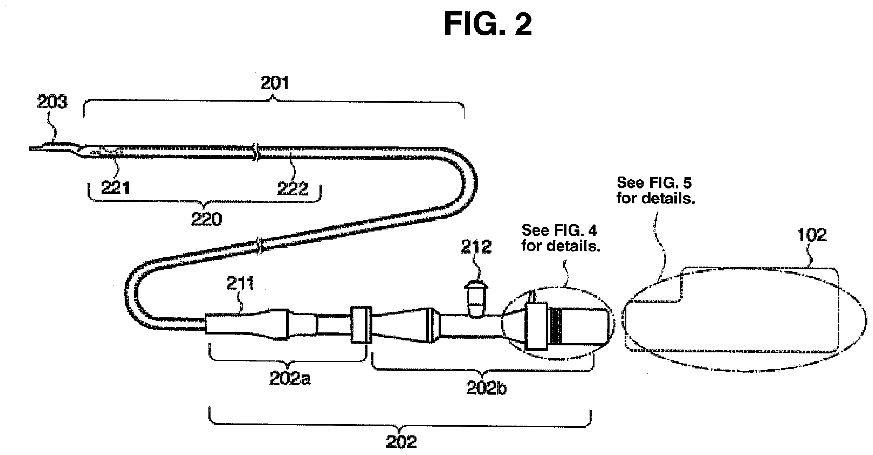

Next, it will be explained with respect to the whole constitution of the probe unit 101 by using FIG. 2. As shown in FIG. 2, the probe unit 101 is constituted by a long-sized catheter sheath 201 to be directly inserted into a body cavity such as a blood vessel and the like, and a connector unit 202 which is not inserted inside the body cavity in order to be steered by a user and which is located on the hand-side of a user. The distal end of the catheter sheath 201 is formed with a tube 203 for a guide wire lumen, and the catheter sheath 201 is formed as a lumen which is continuous from a connection portion of the tube 203 for a guide wire lumen toward a connection portion with the connector unit 202.

In the inside of a lumen of a catheter sheath 201, there is inserted an imaging core 220 approximately over the full length of the catheter sheath 201 and the connector unit 202 in which the imaging core 220 includes a compatible transmitting and receiving unit 221 arranged with a transmitting and receiving unit for transmitting and receiving an ultrasound signal and transmitting and receiving unit for transmitting and receiving an optical signal, and a drive shaft 222 transferring the drive force for rotating the above and concurrently, arranged with an electric signal line for transmitting an ultrasound signal and an optical fiber for transmitting an optical signal in the inside thereof.

The connector unit 202 is composed of a sheath connector 202a constituted integrally at the proximal end of the catheter sheath 201 and a drive shaft connector 202b constituted integrally at the proximal end of the drive shaft 222.

An anti-kink protector 311 is provided at a boundary portion between the sheath connector 202a and the catheter sheath 201. Thus, a predetermined rigidity is maintained and it is possible to prevent a bend (kink) caused by a rapid change.

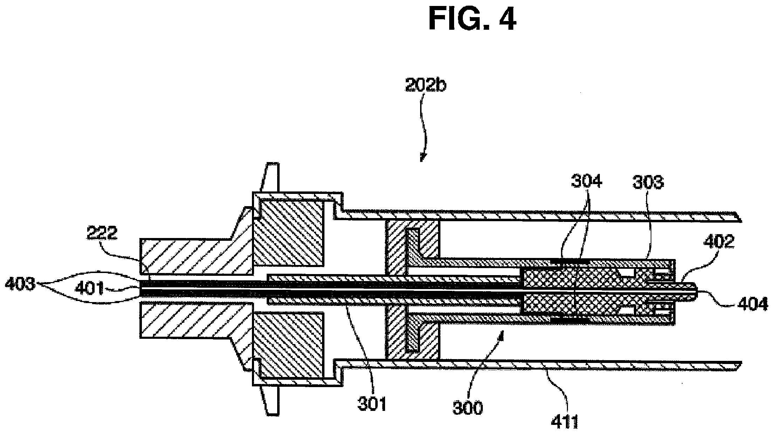

The proximal end side of the drive shaft connector 202b (see FIG. 4 for the details thereof) is constituted so as to be connected detachably with respect to the scanner & pull-back unit 102.

### <3. Sectional Constitution of Imaging Core>

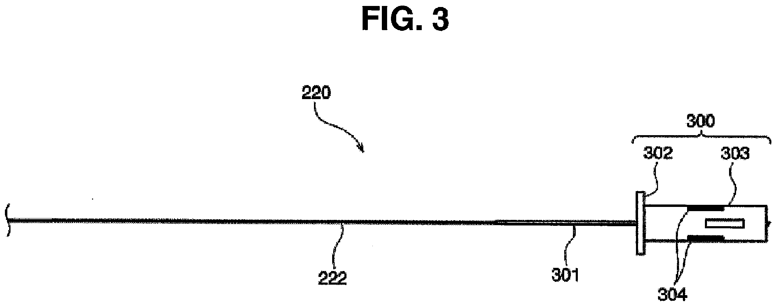

Next, it will be explained with respect to the whole constitution of the imaging core 220. FIG. 3 is a diagram showing a constitution of the proximal end side of the imaging core 220. As shown in FIG. 3, there is attached a connector apparatus 300 on the proximal end side of the imaging core 220. The connector apparatus 300 realizes optical and also electrical connections with respect to an adapter (details thereof are described later) inside the rotational drive unit when the drive shaft connector 202b is connected to the rotational drive unit side of the scanner unit. In addition, it fulfills also a role of transferring the rotationally drive force in the rotational drive unit to the drive shaft 222.

The connector apparatus 300 is located with an APC optical connector (not shown in FIG. 3) in the inside thereof and also, is provided with a connector fixing member 303 arranged with a metal contact point 304 on the circumferential surface thereof and a flange 302 for supporting the connector fixing member 303 freely rotatably in the inside on the proximal end side of the drive shaft connector 202b.

Note that it is assumed that the drive shaft 222 is joined with the APC optical connector located inside the connector apparatus 300 through a connection pipe 301.

### <4. Constitution of Drive Shaft Connector>

Next, it will be explained with respect to the sectional constitution of the drive shaft connector 202b by using FIG. 4. FIG. 4 is a diagram showing an inside constitution on the proximal end side of the drive shaft connector 202b.

As shown in FIG. 4, the connector apparatus 300 is located at a predetermined position on the proximal end side of the drive shaft connector 202b. Also, on the proximal end side of the connector apparatus 300, a connector for optical fiber (APC optical connector) 402 is located and holds an end portion on the proximal end side of the optical fiber 401 provided with a ferrule 404 in the inside of the connector fixing member 303 having a hollow tubular shape. Thus, it becomes a situation in which the optical fiber 401 inside the drive shaft 222 is to be connected optically with the optical adaptor located inside the scanner unit through the APC optical connector 402.

On the other hand, on the outer surface of the connector fixing member 303, there is arranged the metal contact point 304 connected with an electric signal line 403 inside the drive shaft 222. Thus, it becomes a situation in which the electric signal line 403 inside the drive shaft 222 is to be connected electrically with a metal contact point of an adapter fixing member arranged inside the scanner unit.

Note that the connector fixing member 303 includes a disc-shaped flange 302 at the end portion on the distal end side thereof and is held freely rotatably in the inside of a housing 411 of the drive shaft connector 202b.

Note that the connector fixing member 303 is assumed to be constituted so as to carry out the positioning of the APC optical connector 402 toward the circumferential direction in cooperation with the adapter fixing member on an occasion of the coupling with the optical adaptor.

### <5. Inside Constitution of Scanner Unit>

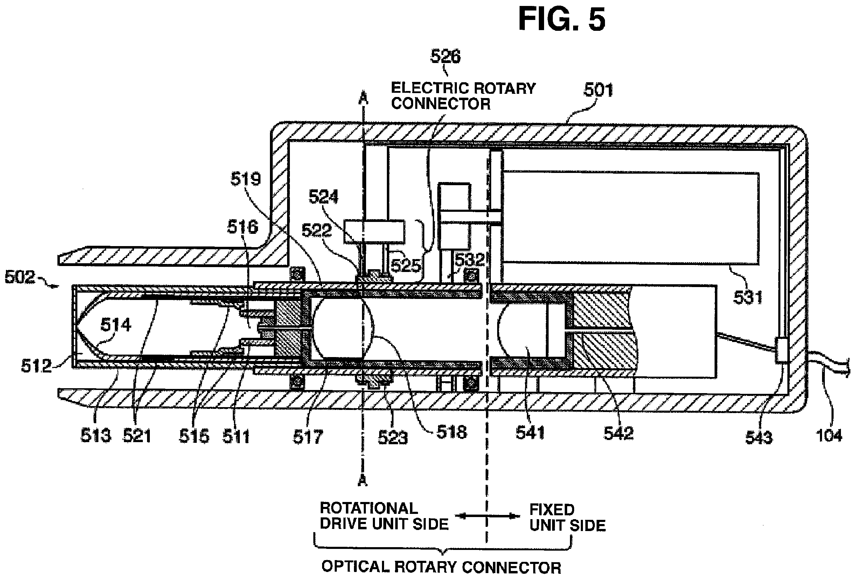

Next, it will be explained with respect to an inside constitution of the scanner unit which constitutes the scanner & pull-back unit 102. FIG. 5 is a diagram showing an inside constitution of the scanner unit.

In FIG. 5, a reference numeral 501 indicates a housing of the scanner unit and it is constituted such that the housing 411 of the drive shaft connector 202b is to be fittable from the opening portion 502 side.

A reference numeral 511 indicates an optical adaptor coupled with the APC optical connector 402. The optical adaptor 511 is formed with a hole 516 having a female type structure which accepts the ferrule 404 of the APC optical connector 402.

A reference numeral 512 indicates an adapter fixing member which is formed by a hollow tubular shape and fixes the optical adaptor 511 in the inside thereof so as not to be rotatable relatively. Note that the adapter fixing member 512 is constituted by a protection tube 513 defining the outer surface and a main body 514 which is fixed on the inner surface of the protection tube 513 and which defines the inner surface of the adapter fixing member 512, and on an occasion of the coupling with the APC optical connector 402, it carries out the positioning of the APC optical connector 402 in the circumferential direction in cooperation with the connector fixing member 303.

On the inner surface of the adapter fixing member 512, there are formed a pair of claws 515 and it becomes a situation in which the APC optical connector 402 is to be integrated tightly with the optical adaptor 511 by a mechanism in which the APC optical connector 402 is engaged with the pair of claws 515.

Further, on the inner surface of the adapter fixing member 512, there are arranged a pair of metal contact points 521 and when the APC optical connector 402 is coupled, electrical connection is realized with respect to the metal contact points 304 arranged on the outer surface of the connector fixing member 303.

A reference numeral 517 indicates a lens fixing sleeve supporting portion and an optical lens 518 is fixed on the inside thereof.

A reference numeral 519 indicates a rotation member, supports the adapter fixing member 512 and the lens fix sleeve supporting portion 517, and concurrently, rotates the adapter fixing member 512 and the lens fixing sleeve supporting portion 517 integrally by receiving the rotationally drive force of a drive motor 531 through a rotational belt 532. Thus, the rotational drive unit side of the scanner unit is formed.

On the circumferential surface of the rotation member 519, metal belts (metal surfaces) 522, 523 are wound around over the whole circumference and between those and brushes 524, 525, there is formed an electric rotary connector (metal contact type rotary connector) 526. Note that the metal belts 522, 523 are connected with the metal contact points 521 respectively and thus, the transmission and reception of the electric signal becomes possible between the electric signal line 403 inside the drive shaft 222 and the brushes 524, 525. Note that it is assumed that the electric rotary connector 526 and the signal line 104 are connected through a connection portion 543.

On the other hand, on the fixed unit side of the scanner unit, there is fixed a lens 541. Thus, it becomes a situation in which the reflected light radiated from the ferrule 404 of the APC optical connector 402 enters into an optical fiber 542 on the fixed unit side in a non-contact manner through the optical lens 518 and the lens 541. Also, it becomes a situation in which the measurement light radiated from the optical fiber 542 enters into the ferrule 404 of the APC optical connector 402 on the rotational drive unit side in a non-contact manner through the lens 541 and the optical lens 518. In other words, these form a non-contact type rotary connector (optical rotary connector). Note that it is assumed that the optical fiber 542 and the signal line 104 are connected through the connection portions 543.

In this manner, the scanner unit includes the metal contact type rotary connector (electric rotary connector) for carrying out transmission of the electric signal between the rotational drive unit and the fixed unit, and the non-contact type rotary connector (optical rotary connector) for carrying out transmission of the optical signal.

### <6. Constitution of Electric Rotary Connector>

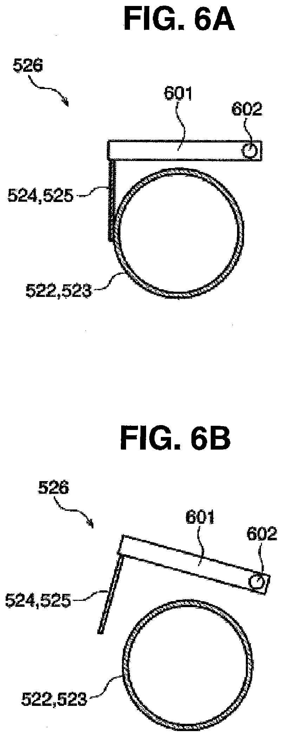

Next, it will be explained with respect to the detailed constitution of the electric rotary connector 526. FIG. 6A and FIG. 6B are diagrams showing vertical cross-section of the electric rotary connector 526 within an A-A cross-section in FIG. 5.

As shown in FIG. 6A and FIG. 6B, in the electric rotary connector 526, a supporting portion 601 for supporting the brushes 524, 525 is mounted on a rotation axis 602 in a freely rotatable manner. Consequently, by operating an operation lever which is not shown and which is provided at the scanner unit, it is possible for the brushes 524, 525 to be operated between a contact position in which they slide on the metal belts 522, 523 and a non-contact position in which they are non-contacted with respect to the metal belts 522, 523. In other words, there is employed such a constitution in which the brushes 524, 525 are switchable between a state in which they contact with the metal belts 522, 523 (state shown in FIG. 6A) and a non-contact state (state shown in FIG. 6B).

In this manner, the electric rotary connector 526 is constituted such that the contact state and the non-contact state between the brushes 524, 525 and the metal belts 522, 523 are switchable, so that in case of carrying out the transmission of the electric signal (that is, in case of using IVUS), it becomes possible to operate the operation lever in the contact state and on the other hand, in case of carrying out the transmission of the optical signal (that is, in case of using OCT or OFDI), it becomes possible to steer the operation lever in the non-contact state.

According to this result, on an occasion of using the OCT or the OFDI, even in case of rotating the rotation member519 high speedily, the wear of the electric rotary connector 526 will never occur and it becomes possible to avoid the breakdown and a cause of short lifetime due to the occurrence of abrasion powders or the like.

### <7. Connection Example between Drive Shaft Connector and Scanner Unit>

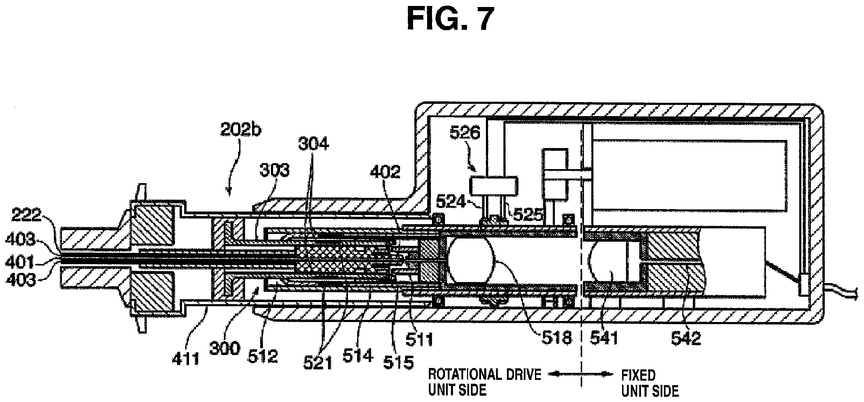

Next, it will be explained with respect to a connection example of the drive shaft connector 202b and the scanner unit. FIG. 7 is a diagram showing a connection example of the drive shaft connector 202b and the scanner unit.

As shown in FIG. 7, when the housing 411 of the drive shaft connector 202b is inserted along the opening portion 502 of the scanner unit, the connector fixing member 303 of the connector apparatus 300 proceeds while sliding on the inner surface of the main body 514 of the adapter fixing member 512, and it is fixed by being engaged with the claw 515.

By a mechanism that the APC optical connector 402 is fixed by the claw 515, the APC optical connector 402 and the optical adaptor 511 are coupled optically and concurrently, the metal contact point 304 and the metal contact point 521 are connected electrically.

Thus, the optical fiber 401 inside the drive shaft 222 and the optical fiber 542 on the fixed unit side are connected in a non-contact manner through the optical lens 518 and the lens 541 and it becomes possible to carry out the transmission of the optical signal between the optical fiber 401 on the rotational drive unit side and the optical fiber 542 on the fixed unit side.

Also, the electric signal line 403 inside the drive shaft 222 and the electric rotary connector 526 are connected through the metal contact points 304, 521 and it becomes possible to carry out the transmission of the electric signal between the electric signal line 403 on the rotational drive unit side and the brushes 524, 525 on the fixed unit side.

As clear from the explanation mentioned above, in the imaging apparatus for diagnosis relating to this illustrative example not falling under the scope of the claims there is employed, in the scanner unit in which the metal contact type rotary connector for the electric signal (electric rotary connector) and the non-contact type rotary connector for the optical signal (optical rotary connector) are located, a constitution in which the electric rotary connector which is a metal contact type rotary connector for the electric signal is switchable between a contact state and a non-contact state.

According to this result, when carrying out the transmission of the optical signal, it becomes possible to switch the electric rotary connector to the non-contact state and it becomes possible to reduce the wear of the electric rotary connector.

### [Second illustrative example not falling under the scope of the claims]

In the first illustrative example not falling under the scope of the claims mentioned above, it was explained with respect to a case in which the electric rotary connector is located on the scanner unit, but the present invention is not limited by this and it is also allowed for the electric rotary connector to be located on the drive shaft connector. Hereinafter, it will be explained with respect to a case in which the electric rotary connector as the metal contact type rotary connector is located on the drive shaft connector.

Note that the outward-appearance constitution of an imaging apparatus for diagnosis, the whole constitution of a probe unit and a sectional constitution of the imaging core and the like are similar as those of the first illustrative example not falling under the scope of the claims mentioned above, so that the explanation will be omitted below.

### <1. Constitution of Drive Shaft Connector>

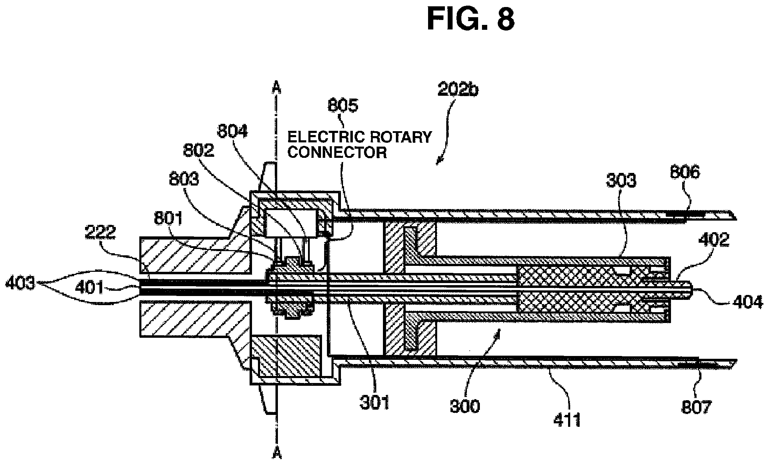

First, it will be explained with respect to an inside constitution on the proximal end side of the drive shaft connector 202b in the imaging apparatus for diagnosis relating to this illustrative example not falling undeer the scope of the claims. FIG. 8 is a diagram showing the inside constitution on the proximal end side of the drive shaft connector 202b in the imaging apparatus for diagnosis relating to this illustrative example not falling undeer the scope of the claims.

As shown in FIG. 8, the connector apparatus 300 is located on the predetermined position on the proximal end side of the drive shaft connector 202b. Also, on the proximal end side of the connector apparatus 300, there is located the connector (APC optical connector) 402 for optical fiber and holds the proximal end side end portion of the optical fiber 401 provided with the ferrule 404 in the inside of the connector fixing member 303 having a hollow tubular shape. Thus, it becomes a situation in which the optical fiber 401 inside the drive shaft 222 is connected with the optical adaptor located inside the scanner unit optically through the APC optical connector 402.

Also, the connector fixing member 303 includes the disc-shaped flange 302 at the distal end side end portion and are held freely rotatably in the inside of the housing 411 of the drive shaft connector 202b.

On the other hand, on the circumferential surface of the connection pipe 301, metal belts 801, 802 are wound around over the whole circumference and there is formed an electric rotary connector (metal contact type rotary connector) 805 between the brushes 803, 804. Note that the metal belts 801, 802 are connected with the electric signal lines 403 respectively and thus, the transmission of the electric signal between the electric signal lines 403 inside the drive shaft 222 and the brushes 803, 804 becomes possible.

Further, on the outer surface of the housing 411 of the drive shaft connector 202b, there are arranged metal contact points 806, 807 and the metal contact points 806, 807 and the electric rotary connector 805 are connected electrically.

In this manner, in case of the imaging apparatus for diagnosis relating to this illustrative example not falling under the scope of the claims there is provided with the metal contact type rotary connector (electric rotary connector) for carrying out the transmission of the electric signal in the inside of the drive shaft connector.

### <2. Constitution of Electric Rotary Connector>

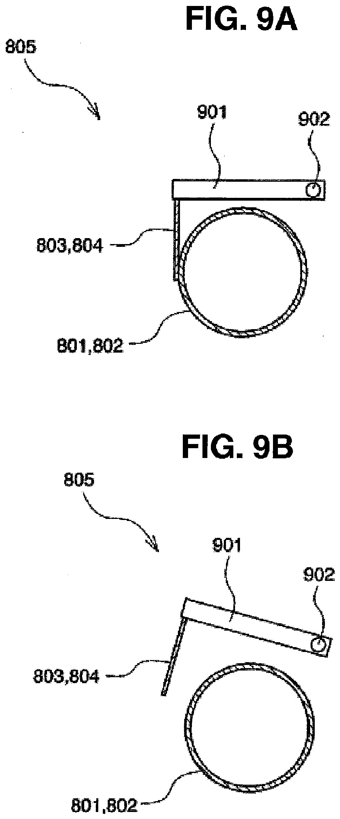

Next, it will be explained with respect to the detailed constitution of the electric rotary connector 805. FIG. 9A and FIG. 9B are views showing the vertical cross-section of the electric rotary connector 805 in an A-A cross-section in FIG. 8.

As shown in FIG. 9A and FIG. 9B, the electric rotary connector 805 is mounted with a supporting portion 901 for supporting the brushes 803, 804 around a rotation axis 902 freely rotatably. Consequently, by operating the operation lever, which is not shown, provided at the drive shaft connector 202b, it is possible for the brushes 803, 804 to be moved between a contact position sliding on the metal belts 801, 802 and a non-contact position which is non-contact with respect to the metal belts 801, 802. In other words, it becomes such a constitution in which it is switchable between a state in which the brushes 803, 804 contact the metal belts 801, 802 (state in FIG. 9A) and a non-contact state (state in FIG. 9B).

In this manner, the electric rotary connector 805 is constituted such that the contact state and the non-contact state between the brushes 803, 804 and the metal belts 801, 802 are switchable, so that in case of carrying out the transmission of the electric signal (that is, in case of using IVUS), the operation lever is operated to the contact state and on the other hand, in case of carrying out the transmission of the optical signal (that is, in case of using OCT or OFDI), it becomes possible to operate the operation lever to the non-contact state.

According to this result, on an occasion of using the OCT or the OFDI, even in case of rotating the rotation member519 high speedily, the wear of the electric rotary connector 805 will never occur and it becomes possible to avoid the breakdown and a cause of short lifetime due to the occurrence of abrasion powders or the like.

### <3. Inside Constitution of Scanner Unit>

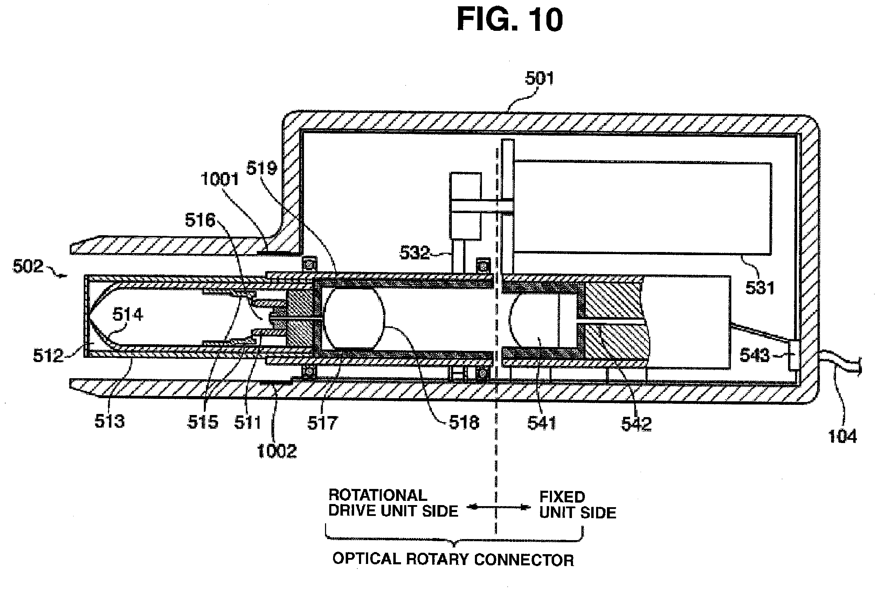

Next, it will be explained with respect to an inside constitution of the scanner unit constituting the scanner & pull-back unit 102. FIG. 10 is a diagram showing an inside constitution of the scanner unit.

In FIG. 10, a reference numeral 501 indicates a housing of the scanner unit, which is constituted such that the housing 411 of the drive shaft connector 202b becomes fittable from the opening portion 502 side.

On the inner surface of the housing 501, metal contact points 1001, 1002 are arranged and connected with metal contact points 806, 807 provided on the outer surface of the housing 411 when the housing 411 of the drive shaft connector 202b is fitted. Note that it is assumed that the metal contact points 1001, 1002 and the signal line 104 are connected through the connection portions 543.

A reference numeral 511 indicates an optical adaptor, which is coupled with the APC optical connector 402. In the optical adaptor 511, there is formed a hole 516 of a female type structure which accepts the ferrule 404 of the APC optical connector 402.

A reference numeral 512 indicates an adapter fixing member, which is composed of a hollow tubular shape and the optical adaptor 511 is fixed in the inside thereof relatively unrotatably. Note that the adapter fixing member 512 is constituted by a protection tube 513 for defining the outer surface and a main body 514 which is fixed on the inner surface of the protection tube 513 and which defines the inner surface of the adapter fixing member 512, and carries out positioning of the circumferential direction of the APC optical connector 402 in cooperation with the connector fixing member 303 when coupling with the APC optical connector 402.

A pair of claws 515 are formed on the inner surface of the adapter fixing member 512 and by engaging the APC optical connector 402 by the pair of claws 515, it becomes a situation in which the APC optical connector 402 is integrated with the optical adaptor 511 tightly.

A reference numeral 517 indicates a lens fixing sleeve supporting portion and an optical lens 518 is fixed in the inside thereof.

A reference numeral 519 indicates a rotation member, which supports the adapter fixing member 512 and the lens fixing sleeve supporting portion 517 and concurrently, rotates the adapter fixing member 512 and the lens fixing sleeve supporting portion 517 integrally by receiving the rotationally drive force of the drive motor 531 through the rotational belt 532. Thus, the rotational drive unit side of the scanner unit is formed.

On the other hand, on the fixed unit side of the scanner unit, the lens 541 is fixed. Thus, it becomes a situation in which the reflected light radiated from the ferrule 404 of the APC optical connector 402 enters into the optical fiber 542 on the fixed unit side in a non-contact manner through the optical lens 518 and the lens 541. Also, it becomes a situation in which the measurement light radiated from the optical fiber 542 enters into the ferrule 404 of the APC optical connector 402 on the rotational drive unit side in a non-contact manner through the lens 541 and the optical lens 518. In other words, these form a non-contact type rotary connector (optical rotary connector). Note that it is assumed that the optical fiber 542 and the signal line 104 are connected through the connection portions 543.

In this manner, the scanner unit is provided with the non-contact type rotary connector (optical rotary connector) for carrying out transmission of the optical signal between the rotational drive unit and the fixed unit.

### <4. Connection Example of Drive Shaft Connector and Scanner Unit>

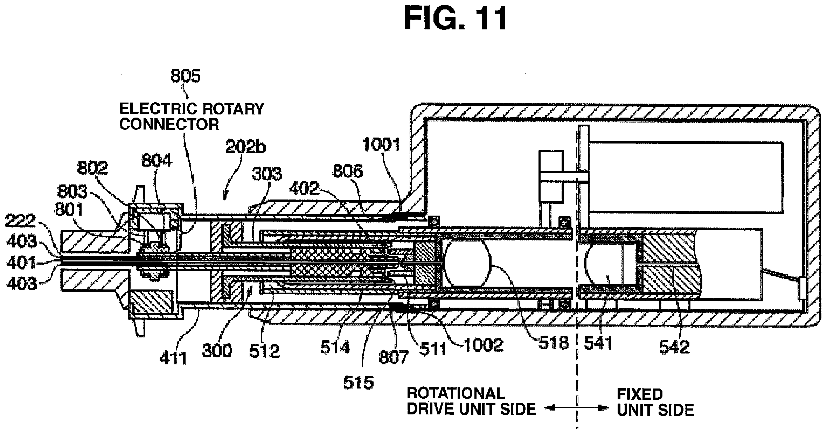

Next, it will be explained with respect to a connection example of the drive shaft connector 202b and the scanner unit. FIG. 11 is a diagram showing an connection example of the drive shaft connector 202b and the scanner unit.

As shown in FIG. 11, when the housing 411 of the drive shaft connector 202b is inserted along the opening portion 502 of the scanner unit, the outer surface of the housing 411 proceeds along the inner surface of the opening portion 502 of the scanner unit and is fixed in a predetermined position.

In this state, it becomes a situation in which the metal contact points 806, 807 arranged on the outer surface of the housing 411 and the metal contact points 1001, 1002 arranged on the inner surface of the scanner unit are connected electrically. Thus, it becomes a state in which the electric signal lines 403 inside the drive shaft 222 and the metal contact points 1001, 1002 are connected through the electric rotary connector 805 and the metal contact points 806, 807 and it becomes possible to carry out transmission of the electric signal between the electric signal lines 403 on the rotational drive unit side and the metal contact points 1001, 1002 on the fixed unit side.

Also, as shown in FIG. 11, when the housing 411 of the drive shaft connector 202b is inserted along the opening portion 502 of the scanner unit, the connector fixing member 303 of the connector apparatus 300 proceeds while sliding on the inner surface of the main body 514 of the adapter fixing member 512 and is fixed by being engaged by the claws 515.

By the fact that the APC optical connector 402 is fixed by the claws 515, it becomes a situation in which the APC optical connector 402 and the optical adaptor 511 are coupled. Thus, the optical fiber 401 inside the drive shaft 222 and the optical fiber 542 on the fixed unit side are connected in a non-contact through the optical lens 518 and the lens 541 and it becomes possible to carry out transmission of the optical signal between the optical fiber 401 on the rotational drive unit side and the optical fiber 542 on the fixed unit side.

As clear from the explanation mentioned above, in the imaging apparatus for diagnosis relating to this illustrative example not falling under the scope of the claims, the metal contact type rotary connector for electric signal (electric rotary connector) is located on the drive shaft connector and concurrently, it was constituted so as to become possible to switch between a contact state and a non-contact state of the metal contact type rotary connector (electric rotary connector) on the drive shaft connector.

According to this result, when carrying out transmission of the optical signal, it becomes possible to switch the electric rotary connector into a non-contact state and it becomes possible to reduce the wear of the electric rotary connector.

### [Third illustrative example not falling under the scope of the claims]

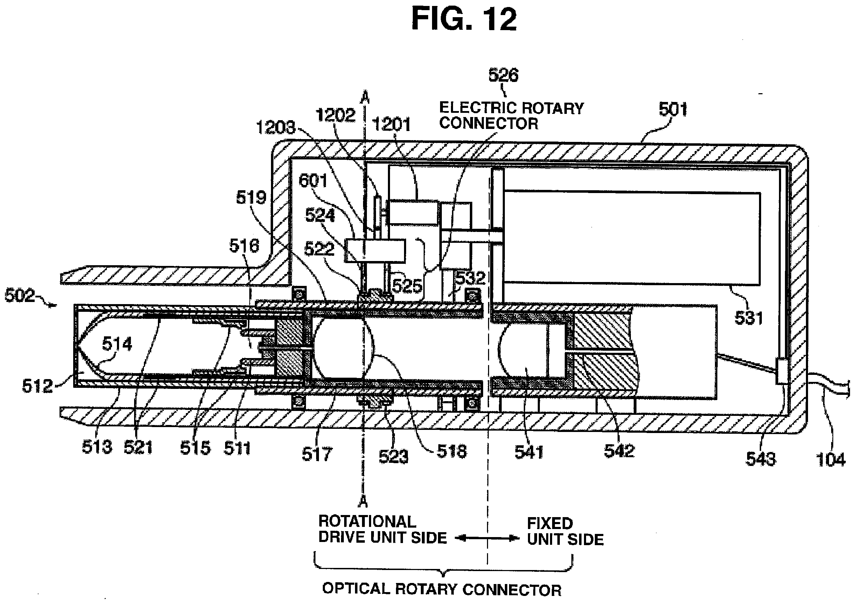

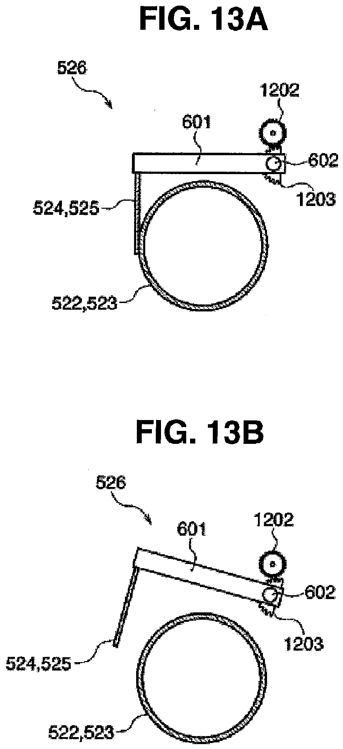

In the first and second illustrative examples not falling under the scope of the claims mentioned above, by a lever operation which is not shown, it was assumed that it is a constitution in which the contact state and the non-contact state of the electric rotary connector are switched, but the present invention is not limited by this and it is allowed to be constituted so as to switch between the contact state and the non-contact state by the electrically-driven by using the motor for brush operation. Hereinafter, it will be explained with respect to a scanner unit switchable between the contact state and the non-contact state of the electric rotary connector by the electrically-driven by using FIG. 12, FIG. 13A and FIG. 13B. Note that the explanation will be carried out centering around the differences from FIG. 5, FIG. 6A and FIG. 6B.

FIG. 12 is a diagram showing an inside constitution of the scanner unit . In FIG. 12, a reference numeral 1201 indicates a motor for brush operation. A gear 1202 is mounted on a shaft of the motor for brush operation 1201 and is coupled with a gear 1203 mounted on the supporting portion 601 for supporting the brushes 524, 525.

Thus, the rotationally drive force of the motor for brush operation 1201 is transmitted to the supporting portion 601, and the supporting portion 601 rotates.

FIG. 13A and FIG. 13B are diagrams showing vertical cross-section of the electric rotary connector 526 within an A-A cross-section in FIG. 12.

As shown in FIG. 13A, the gear 1203 is mounted on the rotation axis 602 of the supporting portion 601, and by the fact that the gear 1202 rotates counterclockwise toward the page face, the supporting portion 601 rotates clockwise around the rotation axis 602 toward the page face. Thus, as shown in FIG. 13B, it is possible to make the brushes 524, 525 be in a non-contact state with respect to the metal belts 522, 523.

As clear from the explanation mentioned above, in the imaging apparatus for diagnosis relating to this illustrative example not falling under the scope of the claims, it was made to be a constitution in which the motor for brush operation is located and a turning direction and a rotation amount of the motor for brush operation are controlled. According to this result, it becomes possible to switch the contact state and the non-contact state of the electric rotary connector by the electrically-driven.

Note that, in this exemplified embodiment, it was explained with respect to a case in which the electric rotary connector is located in the scanner unit, but the present invention is not limited by this aspect and it is needless to say that it is applicable similarly in a case in which the electric rotary connector is located on the drive shaft connector 202b (in case of the second illustrative example not falling under the scope of the claims mentioned above).

### [First Exemplified Embodiment]

In the third illustrative example not falling under the scope of the claims mentioned above, it was explained only with respect to the constitution in which the switching between the contact state and the non-contact state in the electric rotary connector is carried out by the electrically-driven and it was not referred with respect to the details of the operation condition in particular, but in case of the electrically-driven, it becomes possible to cany out various kinds of automatic controls by setting various kinds of operation conditions.

Hereinafter, in this exemplified embodiment, it will be explained with respect to a control method in a case in which the switching between the contact state and the non-contact state in the electric rotary connector is carried out by an automatic control.

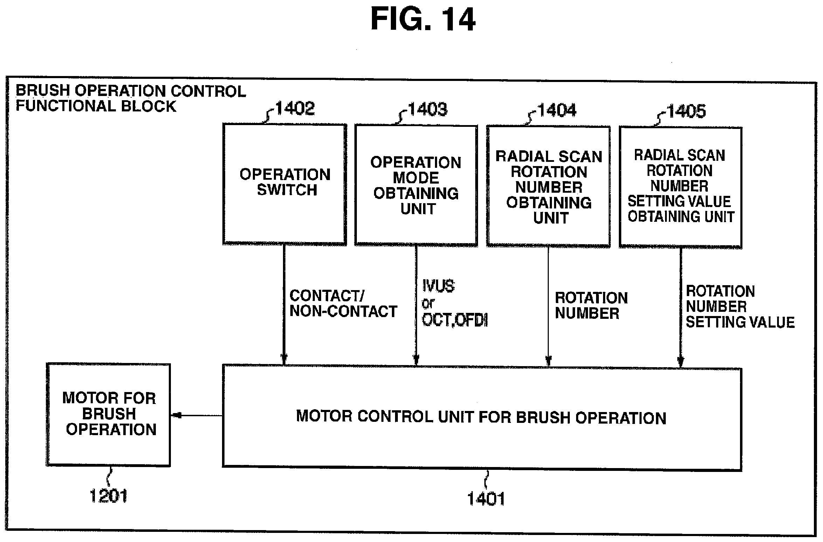

FIG. 14 is one example of a block diagram of a brushing operation control function in the imaging apparatus for diagnosis in order to carry out the switching between the contact state and the non-contact state in the electric rotary connector by an automatic control. It is assumed that the brushing operation control function shown in FIG. 14 can be realized in the operation control apparatus 103 of the imaging apparatus for diagnosis 100.

In FIG. 14, a reference numeral 1401 indicates a motor control unit for brush operation, which controls the turning operation of the motor for brush operation 1201 based on various kinds of operational instructions.

A reference numeral 1402 indicates an operation switch for inputting contact instruction/non-contact instruction and it is assumed that it is located on the operation panel 112. When the contact instruction is inputted through the operation switch 1402, in the control unit for brush operation 1401, the turning direction and the rotation amount of the motor for brush operation 1201 are controlled such that the electric rotary connector 526 becomes in a contact state. Similarly, when the non-contact instruction is inputted through the operation switch 1402, in the control unit for brush operation 1401, the turning direction and the rotation amount of the motor for brush operation 1201 are controlled such that the electric rotary connector 526 becomes in a non-contact state.

A reference numeral 1403 indicates an operation mode obtaining unit, which discriminates whether the own apparatus is the IVUS or the OCT (or OFDI) (classification of imaging apparatus for diagnosis) and inputs the discrimination result to the motor control unit for brush operation 1401.

In a case in which the discrimination result to the effect that the own apparatus is the IVUS is inputted from the operation mode obtaining unit 1403, in the motor control unit for brush operation 1401, the turning direction and the rotation amount of the motor for brush operation 1201 are controlled such that the electric rotary connector 526 becomes in a contact state. On the other hand, in a case in which the discrimination result to the effect that the own apparatus is the OCT (or OFDI) is inputted, in the motor control unit for brush operation 1401, the turning direction and the rotation amount of the motor for brush operation 1201 are controlled such that the electric rotary connector 526 becomes in a non-contact state.

A reference numeral 1404 indicates a radial scan rotation number obtaining unit, which obtains an output of the detection sensor for detecting the rotation number of the drive motor 531 and inputs the rotation number calculated based on the obtained output to the motor control unit for brush operation 1401.

In a case in which the rotation number inputted from the radial scan rotation number setting value obtaining unit 1404 exceeds a predetermined rotation number, in the motor control unit for brush operation 1401, the rotation direction and the rotation amount of the motor for brush operation 1201 are controlled such that the electric rotary connector 526 becomes in a non-contact state. On the other hand, in a case in which the rotation number inputted from the radial scan rotation number setting value obtaining unit 1404 is less than a predetermined rotation number, in the motor control unit for brush operation 1401, the rotation direction and the rotation amount of the motor for brush operation 1201 are controlled such that the electric rotary connector 526 becomes in the contact state.

A reference numeral 1405 indicates a radial scan rotation number set value obtaining unit, which obtains a set value of the radial scan rotation number which is inputted through the operation panel 112, and inputs it to the motor control unit for brush operation 1401.

In a case in which the rotation number inputted from the radial scan rotation number setting value obtaining unit 1405 exceeds a predetermined rotation number, in the motor control unit for brush operation 1401, the rotation direction and the rotation amount of the motor for brush operation 1201 are controlled such that the electric rotary connector 526 becomes in a non-contact state. On the other hand, in a case in which the rotation number inputted from the radial scan rotation number setting value obtaining unit 1405 is less than a predetermined rotation number, in the motor control unit for brush operation 1401, the rotation direction and the rotation amount of the motor for brush operation 1201 are controlled such that the electric rotary connector 526 becomes in a contact state.

As clear from the explanation mentioned above, in the imaging apparatus for diagnosis relating to this exemplified embodiment, it becomes possible to carry out the switching between the contact state and the non-contact state in the electric rotary connector by the automatic control depending on the operation condition of the imaging apparatus for diagnosis.

### [Second Exemplified Embodiment]

In the first to third illustrative examples not falling under the scope of the claims and in the first examplified embodiment mentioned above, it was explained on an assumption that a compatible probe including the transmitting and receiving unit for IVUS and the transmitting and receiving unit for OCT (or OFDI) is connected to the scanner unit. However, the present invention is not limited by this aspect, it is allowed for a probe unit including the transmitting and receiving unit for IVUS or a probe unit including the transmitting and receiving unit for OCT (OFDI) to be connected to the scanner unit independently respectively.

The present invention is not to be limited by the exemplified embodiments described above and it is possible to employ various changes and modifications without departing from the scope of the present invention. Therefore, the following claims are attached in order to open the scope of the present invention.

## Claims

1. An imaging apparatus for diagnosis (100) with which
a first probe provided with a first transmitting and receiving unit for repeating electric signal transmission and reception, and
a second probe provided with a second transmitting and receiving unit for repeating optical signal transmission and reception are configured to be connected detachably, respectively,
said imaging apparatus for diagnosis (100) being configured to obtain a reflected signal inside a body cavity from the first or the second transmitting and receiving unit by rotationally operating the first or the second transmitting and receiving unit inside the body cavity, and which is configured to generate a tomographic image inside the body cavity based on the obtained reflected signal, the imaging apparatus comprising a scanner unit (501) configured to connect detachably with the first probe and the second probe;
an electric rotary connector (526) carrying out transmission of the electric signal with respect to the first transmitting and receiving unit in a state of rotationally operating the first transmitting and receiving unit;
an optical rotary connector carrying out transmission of the optical signal with respect to the second transmitting and receiving unit in a state of rotationally operating the second transmitting and receiving unit, and
wherein the electric rotary connector (526) includes a metal surface mounted on a rotational drive unit side and a brush (524, 525) mounted on a fixed unit side, and the brush (534, 525) is operable between a contact position sliding on the metal surface and a non-contact position which non-contacts with the metal surface,
**characterized in that** the imaging apparatus further comprises a motor (1201) for operating the brush between a contact position and a non-contact position,
and an operation mode obtaining unit (1403) which is configured to discriminate whether the first probe or the second probe is connected to the scanner unit, and to input the discrimination results to the motor control unit for brush operation (1401) which controls the motor (1201) for operating the brush between a contact position and a non-contact position, such that in case the first probe is connected to the scanner unit, the brush is operable in the contact position and in case the second probe is connected to the scanner unit the brush is operable in the non-contact position.

2. The imaging apparatus for diagnosis according to claim 1, **characterized by** further comprising a connection portion (202b) with which the first and second probes are detachably connected respectively, wherein the electric rotary connector and the optical rotary connector are located at the connection portion.

3. The imaging apparatus for diagnosis according to claim 1, **characterized by** further comprising a connection portion (202b) with which the first and second probes are detachably connected respectively, wherein the electric rotary connector is located at the first probe, and the optical rotary connector is located at the connection portion.

## Patentansprüche

1. Bildgebungsvorrichtung für Diagnosezwecke (100), bei der
eine erste Sonde, die mit einer ersten Sende- und Empfangseinheit zum Wiederholen der Übertragung und des Empfangs elektrischer Signale versehen ist; und
eine zweite Sonde, die mit einer zweiten Sende- und Empfangseinheit zum Wiederholen der Übertragung und des Empfangs optischer Signale versehen ist;
so konfiguriert sind, dass sie jeweils lösbar miteinander verbindbar sind;
wobei die Bildgebungsvorrichtung (100) so konfiguriert ist, dass sie innerhalb eines Körperhohlraums von der ersten oder zweiten Sende- und Empfangseinheit bei Drehen der ersten oder zweiten Sende- und Empfangseinheit innerhalb des Körperhohlraums ein reflektiertes Signal erhält, und die Bildgebungsvorrichtung so konfiguriert ist, dass sie basierend auf dem erhaltenen reflektierten Signal ein tomographisches Bild innerhalb des Körperhohlraums erzeugt,
wobei die Bildgebungsvorrichtung Folgendes umfasst:
eine Scannereinheit (501), die so konfiguriert ist, dass sie mit der ersten Sonde und der zweiten Sonde lösbar verbindbar ist;
ein elektrisches Drehverbindungsbauteil (526), das die Übertragung des elektrischen Signals in Bezug auf die erste Sende- und Empfangseinheit in einem Zustand ausführt, in dem die erste Sende- und Empfangseinheit gedreht wird;
ein optisches Drehverbindungsbauteil, das die Übertragung des optischen Signals in Bezug auf die zweite Sende- und Empfangseinheit in einem Zustand ausführt, in dem die zweite Sende- und Empfangseinheit gedreht wird;
wobei das elektrische Drehverbindungsbauteil (526) eine Metalloberfläche aufweist, die auf der Seite der Drehantriebseinheit montiert ist, sowie eine Bürste (524, 525), die auf einer Seite mit einer fest verbauten Einheit montiert ist, und beim Betrieb der Bürste (524, 525) zwischen einer Kontaktposition, bei der die Bürste über die Metalloberfläche gleitet, und einer kontaktlosen Position, bei der die Metalloberfläche von der Bürste unberührt bleibt, gewählt werden kann,
**dadurch gekennzeichnet, dass**
die Bildgebungsvorrichtung ferner Folgendes umfasst:
einen Motor (1201) zum Betreiben der Bürste zwischen einer Kontaktposition und einer kontaktlosen Position, und eine Betriebsartenerfassungseinheit (1403), die so konfiguriert ist, dass sie unterscheidet, ob die erste Sonde oder die zweite Sonde mit der Scannereinheit verbunden ist, und dass die Unterscheidungsergebnisse in die Motorsteuereinheit für den Bürstenbetrieb (1401) eingegeben werden, die den Motor (1201) zum Betreiben der Bürste zwischen einer Kontaktposition und einer kontaktlosen Position steuert, so dass, wenn die erste Sonde mit der Scannereinheit verbunden ist, die Bürste in der Kontaktposition und, wenn die zweite Sonde mit der Scannereinheit verbunden ist, in der kontaktlosen Position betrieben werden kann.

2. Bildgebungsvorrichtung nach Anspruch 1, **dadurch gekennzeichnet, dass** sie ferner Folgendes umfasst:
einen Verbindungsabschnitt (202b), mit welchem die erste und die zweite Sonde jeweils lösbar verbunden sind, wobei das elektrische Drehverbindungsbauteil und das optische Drehverbindungsbauteil am Verbindungsabschnitt angeordnet sind.

3. Bildgebungsvorrichtung nach Anspruch 1, **dadurch gekennzeichnet, dass** sie ferner Folgendes umfasst:
einen Verbindungsabschnitt (202b), mit dem die erste und die zweite Sonde jeweils lösbar verbunden sind, wobei das elektrische Drehverbindungsbauteil an der ersten Sonde und das optische Drehverbindungsbauteil an dem Verbindungsabschnitt angeordnet sind.

## Revendications

1. Dispositif d'imagerie pour diagnostic (100) avec lequel une première sonde équipée d'une première unité d'émission et de réception pour répéter l'émission et la réception de signaux électriques, et une seconde sonde équipée d'une unité d'émission et de réception pour répéter l'émission et la réception de signaux optiques sont configurées pour être connectées de manière amovible, respectivement, ledit dispositif d'imagerie pour diagnostic (100) étant configuré pour obtenir un signal réfléchi à l'intérieur d'une cavité corporelle depuis la première ou la seconde unité d'émission et de réception en faisant fonctionner en rotation la première ou la seconde unité d'émission et de réception à l'intérieur de la cavité corporelle, et qui est configuré pour générer une image tomographique à l'intérieur de la cavité corporelle en fonction du signal réfléchi obtenu, le dispositif d'imagerie comprenant une unité de scanner (501) configurée pour être connectée de manière amovible avec la première sonde et la seconde sonde ;
un connecteur rotatif électrique (526) réalisant l'émission du signal électrique par rapport à la première unité d'émission et de réception dans un état de fonctionnement en rotation de la première unité d'émission et de réception ;
un connecteur rotatif optique réalisant l'émission du signal optique par rapport à la seconde unité d'émission et de réception dans un état de fonctionnement en rotation de la seconde unité d'émission et de réception, et
dans lequel le connecteur rotatif électrique (526) inclut une surface métallique montée sur un côté d'unité d'entraînement en rotation et un balai (524, 525) monté sur un côté d'unité fixe, et le balai (524, 525) peut fonctionner entre une position de contact coulissant sur la surface métallique et une position de non contact qui n'entre pas en contact avec la surface métallique, **caractérisé en ce que** le dispositif d'imagerie comprend en outre un moteur (1201) pour actionner le balai entre une position de contact et une position de non contact, et une unité d'obtention de mode de fonctionnement (1403) qui est configurée pour déterminer si la première sonde ou la seconde sonde est connectée à l'unité de scanner, et pour entrer les résultats de la détermination dans l'unité de commande de moteur pour l'actionnement du balai (1401) qui commande le moteur (1201) pour actionner le balai entre une position de contact et une position de non contact, de telle manière que dans le cas où la première sonde est connectée à l'unité de scanner, le balai peut fonctionner dans la position de contact et dans le cas ou la seconde sonde est connectée à l'unité de scanner le balai peut fonctionner dans la position de non contact.

2. Dispositif d'imagerie pour diagnostic selon la revendication 1, **caractérisé en ce qu'**il comprend en outre une partie de connexion (202b) avec laquelle les premier et second sondes sont connectées de manière amovible respectivement, dans lequel le connecteur rotatif électrique et le connecteur rotatif optique sont situés au niveau de la partie de connexion.

3. Dispositif d'imagerie pour diagnostic selon la revendication 1, **caractérisé en ce qu'**il comprend en outre une partie de connexion (202b) avec laquelle les premier et second sondes sont connectées de manière amovible respectivement, dans lequel le connecteur rotatif électrique est situé au niveau de la première sonde, et le connecteur rotatif optique est situé au niveau de la partie de connexion.
